# FASCICULE DE BREVET EUROPEEN

(11) **EP 2 230 027 B1**
(45) Date de publication et mention de la délivrance du brevet: **08.08.2012**
(21) Numéro de dépôt: 09168871.3
(22) Date de dépôt: 27.08.2009
(51) Int. Cl.: B06B 1/02, H01L 41/04, A61B 17/32, A61C 17/20

(54) **Générateur pour transducteur piézoélectrique**
Generator für einen piezoelektrischen Wandler
Generator for a piezoelectric transducer

(30) Priorité: 16.03.2009 EP 09155282
(43) Date de publication de la demande: 22.09.2010
(73) Titulaire: Tip Top Tips Sàrl, 1180 Rolle (CH)
(72) Inventeur: Denis Klopfenstein, 1110 Morges (CH); Daniel Baour, 1180 Rolle (CH)
(74) Mandataire: Surmely, Gérard

(56) Documents cités:
- FR-A1- 2 391 001
- US-A- 5 543 679
- US-A1- 2001 035 698

## Description

### DOMAINE DE L'INVENTION

La présente invention concerne un générateur pour l'alimentation d'un transducteur piézoélectrique et plus particulièrement un tel générateur utilisable en dentisterie.

### ART ANTERIEUR

On sait que l'impédance d'un transducteur piézoélectrique a tendance à varier considérablement selon les conditions de fonctionnement auxquelles il est soumis. D'une part, l'impédance d'un piézoélectrique atteint son minimum pour la fréquence de résonance série et, d'autre part, une augmentation de la charge mécanique provoque une augmentation de l'impédance. Ces variations d'impédance entraînent certains problèmes. En effet, si le transducteur est alimenté par un générateur du type « source de courant », la puissance délivrée au transducteur est proportionnelle à l'impédance. La puissance peut donc s'accroître de manière incontrôlée. Ainsi, si par exemple l'appareil est utilisé pour le détartrage dentaire, on risque d'attaquer la dent en voulant enlever le tartre. En revanche, si le transducteur est alimenté par un générateur du type « source de tension», la puissance fournie décroît suivant une loi hyperbolique à mesure qu'augmente l'impédance. Ainsi, plus le travail mécanique exigé est important, moins le générateur fournit de puissance ; ce qui n'est pas vraiment idéal. D'autre part, lorsque aucune charge mécanique n'est présente, la puissance appliquée au transducteur s'accroît de manière incontrôlée, ce qui peut se traduire par une rupture de l'outil piézoélectrique.

Le document de brevet FR 2'391'001 propose une solution au problème ci-dessus. Le générateur pour l'alimentation d'un transducteur piézoélectrique décrit dans ce document comporte un transformateur dont le primaire est excité par un oscillateur et dont le secondaire est connecté pour alimenter le transducteur. L'oscillateur lui-même est alimenté en parallèle par un générateur de courant constant (source de courant) et un générateur de tension (source de tension). Des moyens sont prévus pour bloquer le générateur de tension tant que l'impédance de la charge reste inférieure à seuil réglable et, au contraire, pour bloquer le générateur de courant dès que l'impédance de la charge dépasse le seuil réglable.

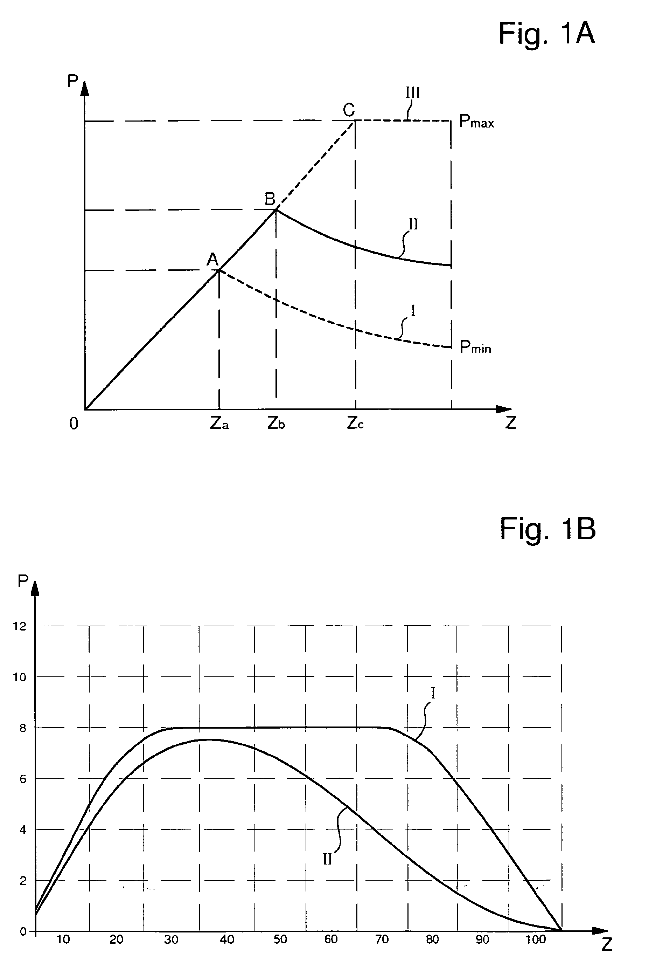

La figure 1A, tirée du document antérieur susmentionné, est un diagramme montrant la puissance délivrée P en fonction de l'impédance Z du transducteur, respectivement dans le cas d'une puissance minimale (courbe I), d'une puissance intermédiaire (courbe II), et de la puissance maximale (courbe III). Si on considère la courbe II correspondant à la puissance intermédiaire, on voit que tant que l'impédance Z du transducteur demeure inférieure au seuil Zb, la puissance P délivrée au transducteur augmente proportionnellement à l'impédance. Si l'impédance dépasse le seuil de référence, le générateur de courant constant est bloqué et le générateur de tension débloqué. Dès cet instant, la puissance délivrée diminue selon une loi hyperbolique à mesure que l'impédance croît.

Le générateur d'alimentation d'un transducteur piézoélectrique qui vient d'être décrit à l'inconvénient de ne fournir une puissance maximale que pour une valeur bien précise de l'impédance du transducteur (comme en témoigne la forme généralement triangulaire des courbes I et II de la figure 1A).

La figure 7 du document US2001/0035698 montre notamment un circuit d'alimentation du primaire d'un transformateur piézoélectrique. Ce circuit comporte d'une part deux transformateurs à induction comprenant chacun un enroulement primaire et un enroulement secondaire, et d'autre part deux switchs commandés par un oscillateur de fréquence ultrasonore de façon à alimenter alternativement les primaires des deux transformateurs à induction. Les secondaires des deux transformateurs à induction sont respectivement reliés aux deux entrées du transformateur piézoélectrique, de manière à lui fournir deux tensions en opposition de phase. On comprendra que la puissance fournie par un tel circuit varie considérablement avec l'impédance de la charge piézoélectrique.

### RESUME DE L'INVENTION

Un but de la présente invention est de fournir un générateur délivrant une puissance sensiblement constante sur un intervalle de variation de l'impédance (comme illustré par la forme généralement trapézoïdale de la première courbe de la figure 1 B). La présente invention atteint ce but en fournissant un générateur pour l'alimentation d'un transducteur piézoélectrique selon la revendication 1 annexée.

### BREVES DESCRIPTION DES FIGURES

D'autres caractéristiques et avantages de la présente invention apparaîtront à la lecture de la description qui va suivre, donnée uniquement à titre d'exemple non limitatif, et faite en référence aux dessins annexés dans lesquels :
- la figure 1A est un est un graphique montrant la puissance délivrée par un générateur de l'art antérieur en fonction de l'impédance du transducteur piézoélectrique ;
- la figure 1 B est un graphique montrant la puissance délivrée par un générateur selon la présente invention en fonction de l'impédance du transducteur piézoélectrique ;
- la figure 2 est un schéma électrique d'un mode de réalisation de l'invention ;
- les figures 3A, 3B et 3C sont des graphiques montrant le courant et la tension dans les enroulements des transformateurs du générateur de la figure 2 pour trois valeurs de l'impédance du transducteur piézoélectrique.

### DESCRIPTION DETAILLEE D'UN MODE DE REALISATION

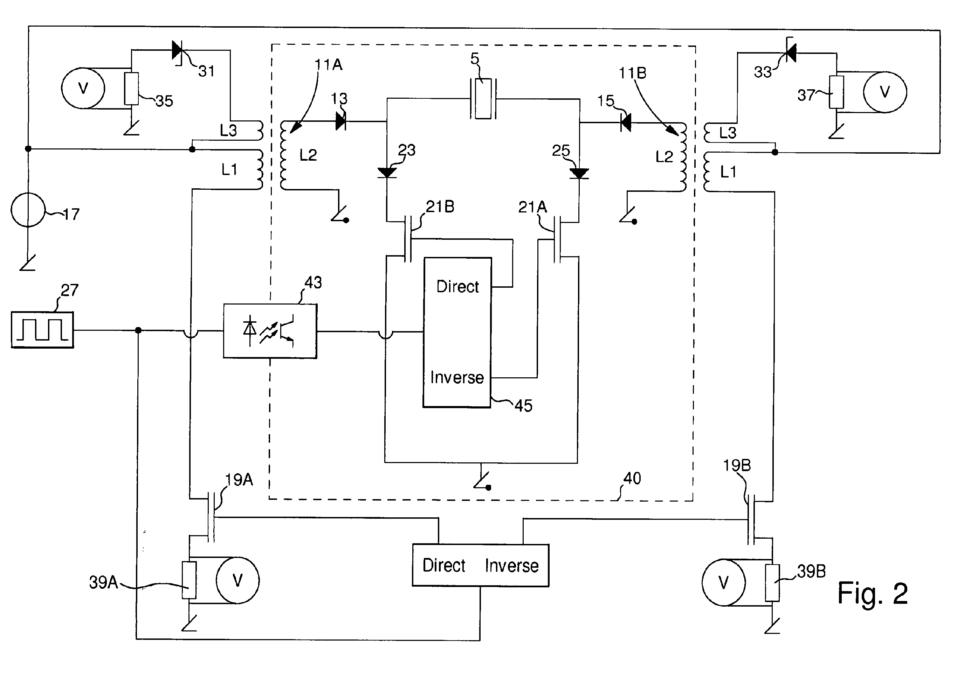

La figure 2 est un schéma électrique d'un générateur selon un mode particulier de réalisation de l'invention. Le générateur pour l'alimentation d'un transducteur piézoélectrique 5 comporte deux transformateurs 11A et 11B comprenant chacun un enroulement primaire L1 et un enroulement secondaire L2. Chacun des deux enroulements secondaires L2 est relié par une de ses bornes à l'un des deux pôles du piézoélectrique 5, une diode (13 ou 15) étant encore intercalée entre chaque enroulement secondaire et le piézoélectrique 5. L'autre borne de chacun des enroulements secondaires L2 est reliée à la masse.

Chacun des enroulements primaires L1 des transformateurs 11A et 11B est connecté, en série avec un interrupteur (19A ou 19B), entre une source de tension 17 et la masse. Les interrupteurs 19A et 19B, tout comme les autres interrupteurs mentionnés dans la présente description, sont des interrupteurs à commande électrique implémentés sous forme de transistors. De tels interrupteurs seront désignés ci-après par leur nom anglais « switch ». En plus d'être reliés aux secondaires L2 des deux transformateurs, les deux pôles du transducteur 5 sont également reliés à la masse par l'intermédiaire d'une diode 23 et d'un switch 21B, respectivement d'une diode 25 et d'un switch 21A. Cela revient à dire, d'une part, que le piézo 5 est connecté, en série avec la diode 13, la diode 25 et le switch 21A, entre les bornes du secondaire L2 du transformateur 11A, et que, d'autre part, le piézo 5 est également connecté, en série avec la diode 15, la diode 23 et le switch 21B, entre les bornes du secondaire L2 du transformateur 11B.

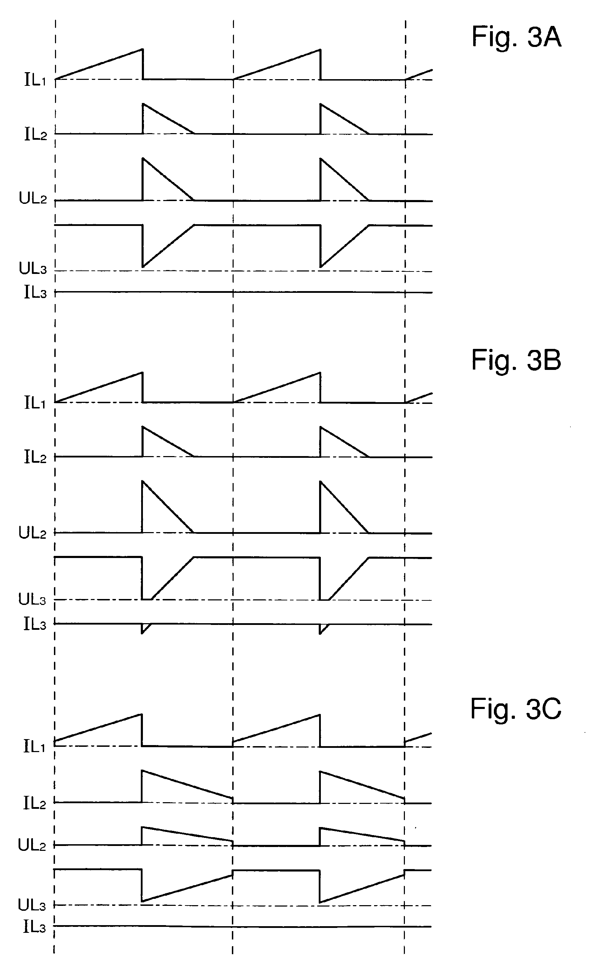

Les switchs 19A, 19B, 21A, 21B sont tous les quatre commandés par le même oscillateur 27 fournissant une tension carrée formée d'une suite d'alternance positive et négative. Pendant les alternances positives, les switchs 19A et 21B sont ouverts et les switchs 19B et 21A sont fermés. Pendant les alternances négatives, c'est l'inverse. Durant les alternances positives, le circuit formé par le secondaire L2 du transformateur 11A, le piézo 5, les diodes 13 et 25 et le switch 21A est fermé et l'énergie stockée dans le transformateur 11A se transfère à la charge. Simultanément, le switch 19B est fermé et le primaire L1 du transformateur 11B est relié directement à la source de tension 17. Le courant à travers le primaire produit une augmentation du flux magnétique. De l'énergie est ainsi stockée dans le circuit magnétique. Durant les alternances négatives, c'est l'inverse. Le secondaire L2 du transformateur 11B restitue son énergie en se déchargeant dans le circuit comprenant le piézo 5, les diodes 15 et 23 et le switch 21B, alors que le courant à travers le primaire L1 du transformateur 11A induit le stockage d'énergie dans son circuit magnétique. Le graphique de la figure 3A montre le comportement du courant et de la tension dans les enroulements L1, L2 et L3 d'un des deux transformateurs 11A ou 11B. On voit que le courant IL1 dans le primaire de transformateur croît régulièrement pendant une alternance avant de retomber à zéro et d'y rester pour la durée de l'alternance suivante. A la transition entre deux alternances, l'enroulement secondaire prend le relais. On voit qu'un courant IL2, dont l'intensité va en diminuant, circule dans le secondaire. Dans l'exemple illustré, le courant IL2 circule jusqu'à dissipation complète de l'énergie stockée. Les variations de la tension UL2 entre les bornes de l'enroulement secondaire accompagnent celles de l'intensité de IL2.

On comprendra que le fait d'avoir deux transformateurs 11A et 11B et de connecter le piézo 5 alternativement à l'un puis à l'autre de ces derniers, permet de fournir au piézo une tension d'alimentation alternative. D'autre part, l'homme du métier appréciera, qu'en résumé, la fonction des switchs 19A, 19B, 21A, 21B est de commander les transformateurs 11A et 11B pour les faire fonctionner sur le mode communément appelé « flyback ».

On voit encore sur la figure 2 que deux résistances (référencées respectivement 39A et 39B) sont connectées en série avec les enroulements primaires L1 et les interrupteurs 19A et 19B. Les résistances 39A et 39B sont respectivement prévues pour mesurer l'intensité du courant circulant dans les primaires L1 des transformateurs 11A et 11B. La mesure du courant dans L1 est prévue de façon à permettre la détection d'un maximum de l'intensité du courant. Ce maximum indiquant que le piézo 5 vibre à sa fréquence de résonance. En effet, comme on l'a déjà dit, l'impédance d'un piézoélectrique atteint son minimum à la fréquence de résonance. Ainsi, à la fréquence de résonance, la charge connectée entre les bornes du secondaire L2 est minimale. Dans ces conditions, il peut arriver que la charge soit insuffisante pour dissiper intégralement l'énergie stockée dans le transformateur. Cette situation est illustrée par le graphique de la figure 3C. En se référant à ce graphique, on peut voir que, lorsque la charge est particulièrement basse, le courant IL2 et la tension UL2 n'ont pas le temps de retomber à zéro avant la fin d'une alternance. On peut voire également que l'énergie non dissipée dans le secondaire se retrouve dans le primaire au début de l'alternance suivante. Cette énergie non dissipée est responsable de l'intensité non nulle du courant IL1 dans l'enroulement primaire L1 en début d'alternance (Figure 3C). On comprendra donc qu'en dessous d'un certain seuil, plus l'impédance est petite, plus l'intensité du courant IL1 dans le primaire est grande. Il s'en suit qu'un courant IL1 d'intensité maximale indique que le transducteur vibre à la résonance. La mesure du courant IL1 traversant les résistances 39A et 39B peut de manière avantageuse être utilisée dans une boucle de rétroaction (non représentée) pour commander l'oscillateur 27 de manière à ce qu'il oscille à la fréquence de résonance du piézo 5.

On peut voire encore sur la figure 2 que les transformateur 11A et 11B comportent encore chacun un enroulement tertiaire L3. L'enroulement L3 du transformateur 11A est connecté, en série avec une diode 31 et une résistance 35, entre la source de tension 17 et la masse. De façon analogue, l'enroulement L3 du transformateur 11B est connecté, en série avec diode 33 et une résistance 37, entre la source de tension 17 et la masse. Comme on va le voir plus en détail ci-après, la fonction des enroulements L3 est de limiter la tension maximale fournie en sortie du secondaire L2.

La vitesse avec laquelle l'intensité du courant dans L2 diminue lorsque l'énergie stockée dans un des transformateurs est transférée à la charge dépend naturellement de l'impédance associée à la charge. Plus cette impédance est élevée, plus l'intensité du courant décroît rapidement, et plus la tension entre les bornes de l'enroulement secondaire est élevée. Le graphique de la figure 3B décrit le comportement du générateur de la figure 2 dans une situation où la charge mécanique sur le transducteur piézoélectrique 5 est particulièrement élevée. On peut voir sur la figure 3B que l'intensité du courant IL2 décroît sensiblement plus rapidement que dans la figure 3A. De plus, la tension UL2 au début d'une alternance est également considérablement plus élevée que dans le cas de la figure 3A. On comprendra que si, pour une raison ou une autre, la charge mécanique sur le piézo s'élève de manière incontrôlée, la tension en sortie de UL2 risque de s'accroître au point d'endommager le générateur. C'est la raison pour laquelle, dans le présent exemple, les deux transformateurs 11A et 11B comportent chacun un troisième enroulement L3 qui est couplé inductivement avec les enroulements primaire et secondaire L1 et L2.

En se référant à nouveau à la figure 2, on voit que les diodes 31 et 33 sont connectées aux enroulements L3 par leur cathode, et elles sont connectées à la masse par leur anode. Comme l'autre borne de chaque enroulement L3 est connectée au pôle positif de la source de tension 17, les diodes sont normalement soumises à une tension UL3 négative. Dans ces conditions, les diodes 31 et 33 empêchent le passage du courant. Toutefois, si la tension induite dans L3 dépasse la tension d'alimentation continue, la tension UL3 résultante sur les diodes devient transitoirement positive, et un courant IL3 se met à circuler dans L3. Ce courant IL3 transitoire a pour effet de limiter la tension UL2 aux bornes de l'enroulement L2. La présence de l'enroulement L3 permet donc de plafonner la tension UL2 à une valeur qui est déterminée par le choix du rapport entre les valeurs des inductions L2 et L3.

La figure 1B est un graphique comportant une première courbe qui montre le comportement de la puissance fournie par le générateur qui vient d'être décrit en fonction de l'impédance du transducteur piézoélectrique. Le graphique comporte également une deuxième courbe qui correspond au comportement de la puissance d'un générateur de l'art antérieur comme celui décrit dans le document de brevet FR 2'391'001 déjà mentionné. On voit sur la figure 1B que la première courbe comporte une première portion croissante, un deuxième portion constante et enfin une troisième portion décroissante. La deuxième portion occupe toute la partie centrale du graphique et correspond donc à des valeurs moyennes de l'impédance. Dans cet intervalle, la puissance fournie par le générateur selon l'invention est sensiblement constante, et le comportement du générateur correspond à ce qui est décrit par le graphique de la figure 3A. La première portion de la courbe correspond à des valeurs de l'impédance qui ne sont pas suffisantes pour entièrement dissiper l'énergie stockée dans les transformateurs avant la fin d'une alternance. Cette première portion de la courbe correspond à un intervalle dans lequel le comportement du générateur correspond à ce qui est décrit par le graphique de la figure 3C. Dans cet intervalle, la puissance fournie se réduit en proportion de l'impédance. La troisième portion de la courbe correspond aux impédances les plus élevées. Le comportement du générateur dans cette zone correspond à ce qui est décrit par le graphique de la figure 3B. Dans cette zone, la tension entre les bornes du secondaire L2 est limitée par l'enroulement L3 et le courant décroît donc à mesure que l'impédance croît.

Un autre avantage de la présente invention est que les transformateurs 11A et 11B permettent d'isoler galvaniquement le transducteur piézoélectrique 5 de l'alimentation 17. Sur la figure 2, on a représenté par un rectangle en traits interrompus 40 la séparation du générateur en deux zones isolées galvaniquement l'une de l'autre. On peut voir encore sur la figure 2 que les switchs 21A et 21B se trouvent à l'intérieur du rectangle en traits interrompus. Pour assurer l'isolation galvanique au niveau de la commande des switchs, un optocoupleur 43 est intercalé entre l'oscillateur 27 et un amplificateur 45 prévu pour commander les deux switchs.

## Revendications

1. Générateur pour alimenter un moteur piézoélectrique (5), notamment pour applications médicales ou dentaires, le générateur comportant une alimentation (17), un premier et un second transformateur (11B, 11A) comprenant chacun un enroulement primaire (L1) et un enroulement secondaire (L2), et deux premiers switchs (19A, 19B), l'enroulement primaire (L1) du premier transformateur (11B), l'un des deux premiers switchs (19B) et l'alimentation (17), formant ensemble une première boucle de circuit primaire, et l'enroulement primaire (L1) du second transformateur (11A), l'autre des deux premiers switchs (19A) et l'alimentation (17), formant ensemble une seconde boucle de circuit primaire, les deux premiers switchs étant agencés pour être commandés par des alternances positives et négatives fournies par un oscillateur de fréquence ultrasonore, de manière à fermer et à ouvrir alternativement la première et la seconde boucle de circuit primaire de façon à ce que l'enroulement primaire (L1) du premier transformateur (11B) se charge en énergie pendant l'alternance positive, et que l'enroulement primaire (L1) du second transformateur (11A) se charge en énergie pendant l'alternance négative, **caractérisé en ce que** le générateur comprend deux autres switchs (21A, 21B), l'enroulement secondaire (L2) du premier transformateur (11 B) et l'un des deux autres switchs (21 B) étant prévus pour être connectés avec le moteur piezoélectique (5), de manière à former ensemble avec ce dernier une première boucle de circuit secondaire, et l'enroulement secondaire (L2) du second transformateur (11A) et l'autre des deux autres switchs (21A) étant prévus pour être connectés avec le moteur piezoélectique (5), de manière à former ensemble avec ce dernier une seconde boucle de circuit secondaire, les deux autres switchs (21A, 21 B) étant en outre agencés pour être commandés par lesdites alternances positives et négatives, de manière à fermer et à ouvrir alternativement la première et la seconde boucle de circuit secondaire de façon à ce que l'enroulement secondaire (L2) du second transformateur (11A) se décharge à travers le moteur piézoélectrique (5) pendant l'alternance positive, et à ce que l'enroulement secondaire (L2) du premier transformateur (11B) se décharge à travers le moteur piézoélectrique (5) pendant l'alternance négative.

2. Générateur pour moteur piézoélectrique selon la revendication 1, dans lequel chaque transformateur (11A, 11B) comprend un troisième enroulement (L3) maintenu à une tension fixe et une diode (31, 33) en série avec le troisième enroulement de manière à limiter la tension négative entre les bornes du troisième enroulement (L3).

3. Générateur pour moteur piézoélectrique selon la revendication 1 ou 2, dans lequel au moins un des transformateurs (11A, 11 B) comporte un circuit de mesure (39A, 39B) du courant dans l'enroulement primaire (L1) prévu pour détecter une éventuelle forme trapézoïdale du courant qui serait la manifestation de la présence d'énergie non dissipée subsistant depuis l'alternance précédente.

4. Générateur pour moteur piézoélectrique selon la revendication 2 ou 2 et 3, dans lequel au moins un des transformateurs (11A, 11 B) comporte un circuit de mesure (35, 37) du courant dans le troisième enroulement (L3), prévu pour détecter si le troisième enroulement laisse passer un courant, et donc de détecter si la tension négative entre les bornes du troisième enroulement a été limitée.

## Claims

1. A generator for supplying a piezoelectric motor (5), in particular for medical or dental practice, the generator comprising a power supply (17), a first and a second transformer (11B, 11A) each including a primary winding (L1) and a secondary winding (L2), and two first switches (19A, 19B), the primary winding (L1) of the first transformer (11B), one of the two first switches (19B) and the power supply (17), forming together a first primary circuit loop, and the primary winding (L1) of the second transformer (11A), the other one of the two first switches (19A) and the power supply (17), forming together a second primary circuit loop, the two first switches being arranged to be controlled by positive and negative half-cycles provided by an ultrasonic frequency oscillator, in such a way as to close and open alternately the first and the second primary circuit loops so that the primary winding (L1) of the first transformer (11B) is charged with power during the positive half-cycle, and the primary winding (L1) of the second transformer (11A) is charged with power during the negative half-cycle, **characterised in that** the generator comprises two other switches (21A, 21 B), the secondary winding (L2) of the first transformer (11 B) and one of the two other switches (21 B) being adapted to be connected with the piezoelectric motor (5) in such a way as to form together with the latter a first secondary circuit loop, and the secondary winding (L2) of the second transformer (11A) and the other one of the two other switches (21A) being adapted to be connected with the piezoelectric motor (5) in such a way as to form together with the latter a second secondary circuit loop, the two other switches (21A, 21B) further being arranged to be controlled by said positive and negative half-cycles, in such a way as to close and open alternately the first and the second secondary circuit loops so that the secondary winding (L2) of the second transformer (11A) is discharged through the piezoelectric motor (5) during the positive half-cycle, and so that the secondary winding (L2) of the first transformer (11B) is discharged through the piezoelectric motor (5) during the negative half-cycle.

2. The generator for supplying a piezoelectric motor (5) according to claim 1, in which each transformer (11 A, 11 B) comprises a third winding (L3) maintained at a fixed voltage and a diode (31, 33) in series with the third winding so as to limit the negative voltage between the terminals of the third winding (L3).

3. The generator for supplying a piezoelectric motor (5) according to claim 1 or 2, in which at least one of the first and second transformers (11A, 11B) comprises a circuit (39A, 39B) for measuring the current in the primary winding (L1) for making it possible to monitor the shape of the current in such a way as to detect a possible trapezoidal shape of the current caused by the presence of non dissipated energy remaining from the preceding half-cycle.

4. The generator for supplying a piezoelectric motor (5) according to claim 2 or 2 and 3, in which at least one of the first and second transformers (11A, 11B) comprises an additional circuit (35, 37) for measuring the current in the third winding (L3) for making it possible to detect if a current runs through the third winding, and thus to detect if the negative voltage between the terminals of the third winding has been limited.

## Patentansprüche

1. Generator zum Versorgen eines piezoelektrischen Motors (5), insbesondere für medizinische oder zahnärztliche Anwendungen, wobei der Generator eine Versorgung (17), einen ersten und einen zweiten Transformator (11B, 11A) mit jeweils einer Primärwicklung (L1) und einer Sekundärwicklung (L2) und zwei erste Schalter (19A, 19B) umfasst, wobei die Primärwicklung (L1) des ersten Transformators (11B), einer der zwei ersten Schalter (19B) und die Versorgung (17) gemeinsam eine erste Primärschaltungsschleife bilden und die Primärwicklung (L1) des zweiten Transformators (11A), der andere der zwei ersten Schalter (19A) und die Versorgung (17) gemeinsam eine zweite Primärschaltungsschleife bilden, wobei die zwei ersten Schalter so eingerichtet sind, dass sie durch positive und negative Halbperioden gesteuert werden, die durch einen Ultraschall-Frequenzoszillator geliefert werden, um abwechselnd die erste und die zweite Primärschaltungsschleife zu schließen und zu öffnen, so dass sich die Primärwicklung (L1) des ersten Transformators (11 B) während der positiven Halbperiode mit Energie auflädt, und dass sich die Primärwicklung (L1) des zweiten Transformators (11A) während der negativen Halbperiode mit Energie auflädt, **dadurch gekennzeichnet, dass** der Generator zwei weitere Schalter (21A, 21 B) umfasst, wobei die Sekundärwicklung (L2) des ersten Transformators (11 B) und einer der zwei weiteren Schalter (21 B) so vorgesehen sind, dass sie mit dem piezoelektrischen Motor (5) verbunden werden, um gemeinsam mit diesem letzteren eine erste Sekundärschaltungsschleife zu bilden, und die Sekundärwicklung (L2) des zweiten Transformators (11A) und der andere der zwei weiteren Schalter (21A) so vorgesehen sind, dass sie mit dem piezoelektrischen Motor (5) verbunden werden, um gemeinsam mit diesem letzteren eine zweite Sekundärschaltungsschleife zu bilden, wobei die zwei weiteren Schalter (21A, 21 B) außerdem so eingerichtet sind, dass sie durch die positiven und negativen Halbperioden gesteuert werden, um abwechselnd die erste und die zweite Sekundärschaltungsschleife zu schließen und zu öffnen, so dass sich die Sekundärwicklung (L2) des zweiten Transformators (11A) über den piezoelektrischen Motor (5) während der positiven Halbperiode entlädt und sich die Sekundärwicklung (L2) des ersten Transformators (11B) über den piezoelektrischen Motor (5) während der negativen Halbperiode entlädt.

2. Generator für einen piezoelektrischen Motor nach Anspruch 1, wobei jeder Transformator (11A, 11B) eine dritte Wicklung (L3), die auf einer festen Spannung gehalten wird, und eine Diode (31, 33) in Reihe mit der dritten Wicklung umfasst, um die negative Spannung zwischen den Anschlüssen der dritten Wicklung (L3) zu begrenzen.

3. Generator für einen piezoelektrischen Motor nach Anspruch 1 oder 2, wobei mindestens einer der Transformatoren (11A, 11B) eine Schaltung (39A, 39B) zum Messen des Stroms in der Primärwicklung (L1) umfasst, die vorgesehen ist, um eine eventuelle Trapezform des Stroms zu detektieren, die die Offenbarung der Anwesenheit von nicht abgeleiteter Energie wäre, die seit der vorangehenden Halbperiode fortbesteht.

4. Generator für einen piezoelektrischen Motor nach Anspruch 2 oder 2 und 3, wobei mindestens einer der Transformatoren (11A, 11 B) eine Schaltung (35, 37) zum Messen des Stroms in der dritten Wicklung (L3) umfasst, die vorgesehen ist, um zu detektieren, ob die dritte Wicklung einen Strom durchlässt, und folglich zu detektieren, ob die negative Spannung zwischen den Anschlüssen der dritten Wicklung begrenzt wurde.
